# EUROPEAN PATENT APPLICATION

(11) **EP 4 699 694 A1**
(43) Date of publication of application: **25.02.2026**
(21) Application number: 24880137.5
(22) Date of filing: 17.10.2024
(51) Int. Cl.: B01J 35/39, B01J 27/08, B01J 23/70, B01D 53/86, C01B 32/40, C01B 3/58

(54) **PHOTOCATALYST FOR CAPTURING AND CONVERTING LOW CONCENTRATION CARBON DIOXIDE IN AIR**

(30) Priority: 18.10.2023 KR 20230139467
(71) Applicant: Kyungpook National University Industry-Academic Cooperation Foundation, Daegu 41566 (KR)
(72) Inventor: PARK, Hyun Woong, Daegu 41950 (KR); PARK, Ji Yeon, Jinju-si Gyeongsangnam-do 52704 (KR)
(74) Representative: Lewis Silkin LLP
(86) International application number: PCT/KR2024/015744
(87) International publication number: WO 2025/084800

(57) **Abstract**

Disclosed are a photocatalyst for capturing and converting low-concentration carbon dioxide in the air, and a method for capturing and photo-converting carbon dioxide in the air using same. The photocatalyst for capturing and converting carbon dioxide may comprise bismuth oxyhalide in which at least one metal is supported and which has oxygen vacancies.

## Description

### FIELD

The present disclosure relates to a photocatalyst for capturing and converting carbon dioxide capable of directly capturing low concentration carbon dioxide under actual atmospheric conditions and converting the captured carbon dioxide into a high value-added material, and a method for capturing and photo-converting carbon dioxide in the atmosphere using the photocatalyst.

### DESCRIPTION OF RELATED ART

It is predicted that a carbon dioxide emission will continue to increase, and a global temperature will rise by 2.1°C by 2100. In order to solve this problem, research on technology development for carbon dioxide reduction is actively underway, but in reality, it is difficult to rapidly reduce the carbon dioxide emission within a short period of time. As a solution for sustainable growth, carbon capture technology (CCS) or CCU technology is currently adopted as a realistic scheme for carbon dioxide reduction, and direct air capture (Direct air CO₂ Capture) (DAC) technology is being demonstrated.

However, such a technique mainly uses metal-organic frameworks (MOFs), zeolites, and the like as a carbon dioxide capturing agent. In this regard, water resource consumption and additional energy consumption such as heat for regeneration of a capture material is raised as a problem. In addition, due to the competitive adsorption manner, oxygen and moisture contained in the actual air are greatly affected, and the durability of MOFs is weakened at high temperatures. Thus, when this is applied to a reaction of continuously removing carbon dioxide from the actual atmosphere, this may be problematic.

### SUMMARY OF DISCLOSURE

### TECHNICAL PURPOSE

One purpose of the present disclosure is to provide a photocatalyst for capturing and converting carbon dioxide capable of selectively capturing low concentration carbon dioxide in the atmosphere under a condition containing oxygen and moisture and converting the captured carbon dioxide into a high value-added compound using sunlight.

Another purpose of the present disclosure is to provide a method for capturing and photo-converting carbon dioxide in the atmosphere using the photocatalyst.

### TECHNICAL SOLUTION

In one aspect, the present disclosure provides a photocatalyst for capturing and converting carbon dioxide, the photocatalyst comprising bismuth oxyhalide one or more metals supported thereon and having an oxygen vacancy.

In an embodiment, the metal may be selected from Cu, Fe, Co, Ni, Mn, Ru, Pt, Au, and Ag.

In one embodiment, the metal may be contained at a content of 0.01 to 2 wt% based on a total weight of the photocatalyst.

In an embodiment, the metal may include Cu and Fe, wherein based on the total weight of the photocatalyst, the Cu may be contained at a content of 0.01 to 0.5 wt%, and the Fe may be contained at a content of 0.3 to 1 wt%.

In an embodiment, the photocatalyst may selectively capture carbon dioxide having a concentration of 1000 ppm or lower in the atmosphere under a dark condition, and may convert the captured carbon dioxide into CO or CH₄ upon irradiating light thereto.

In another aspect, the present disclosure provides a method for capturing and photo-converting carbon dioxide in the atmosphere, the method comprising a first step of selectively capturing carbon dioxide in the atmosphere using the photocatalyst, and a second step of irradiating light to the photocatalyst having the carbon dioxide captured thereon to reduce the captured carbon dioxide to a photo-conversion product.

In an embodiment, the first step may be performed under a dark condition.

In an embodiment, the photo-conversion product may include CO or CH₄.

### TECHNICAL EFFECT

The photocatalyst according to the present disclosure has improved carbon dioxide adsorption and catalytic activity due to the oxygen vacancy activity of the bismuth oxyhalide, and may additionally improve the photo-conversion of the captured carbon dioxide due to the structure in which one or more metals are supported thereon.

In addition, using the photocatalyst of the present disclosure, low concentration (about 450 ppm) carbon dioxide in the atmosphere may be selectively adsorbed in the dark condition (night) including oxygen and moisture, and may photo-convert the adsorbed carbon dioxide to be reduced into a high value-added material in a light condition (day).

### BRIEF DESCRIPTION OF DRAWINGS

FIG. 1 is a diagram showing a photocatalyst for capturing and converting carbon dioxide according to an embodiment of the present disclosure.
FIG. 2 is a schematic diagram showing a method for capturing and photo-converting carbon dioxide in the atmosphere according to an embodiment of the present disclosure.
FIGS. 3 to 13 are diagrams showing experimental results according to experimental examples of the present disclosure.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components. In the accompanying drawings, the dimensions of the structures are shown in an enlarged manner for clarity of the present disclosure.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

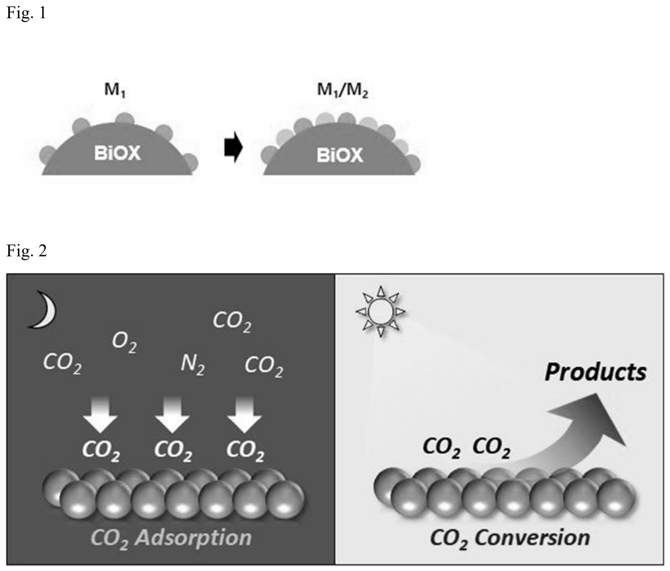

FIG. 1 is a diagram showing a photocatalyst for capturing and converting carbon dioxide according to an embodiment of the present disclosure.

Referring to FIG. 1, the photocatalyst for capturing and converting carbon dioxide according to an embodiment of the present disclosure may comprise bismuth oxyhalide on which one or more metals are supported and which has an oxygen vacancy.

The bismuth oxyhalide of the present disclosure may be represented by BiOX (X=Cl, Br, I). In addition, the bismuth oxyhalide of the present disclosure may have the oxygen vacancy. Bismuth oxyhalide activated using the oxygen vacancy may adsorb carbon dioxide in the atmosphere about three times better than bismuth oxyhalide without the oxygen vacancy.

The bismuth oxyhalide having the oxygen vacancy of the present disclosure may have one or more metals supported thereon. As the metal is supported thereon, the carbon dioxide capture performance of the photocatalyst is improved, and the photo-conversion performance thereof may be improved by about 2.5 times or greater compared to the bismuth oxyhalide activated with the oxygen vacancy.

In an embodiment, the metal may be selected from Cu, Fe, Co, Ni, Mn, Ru, Pt, Au, and Ag. However, a type of the metal is not particularly limited. In addition, in order to improve the selective capturing and photo-conversion performance of low concentration (about 1000 ppm or lower) carbon dioxide in the atmosphere, the metal may be contained at a content of 0.01 to 2 wt% based on the total weight of the photocatalyst. However, the present disclosure is not limited thereto, and the metal may be contained in an appropriate content based on the type of the metal.

Preferably, the photocatalyst may include a bimetallic metal. In this case, the bimetallic metal may include two metals selected from the above-described metals, and most preferably, the bimetallic metal may include Cu and Fe. In addition, in order to obtain the optimal carbon dioxide capturing and photo-conversion performance, the Cu may be contained at a content of 0.01 to 0.5 wt% and the Fe may be contained at a content of 0.3 to 1 wt%, based on the total weight of the photocatalyst.

The photocatalyst of the present disclosure may selectively capture carbon dioxide having a concentration of 1000 ppm or lower in the atmosphere under a dark condition, and may convert the captured carbon dioxide into CO or CH₄ upon irradiating light thereto.

More specifically, the photocatalyst of the present disclosure selectively adsorbs low concentration (about 450 ppm) carbon dioxide in the atmosphere in a dark condition (night) including oxygen and moisture, and photo-converts the adsorbed carbon dioxide in a light condition (day) such that the carbon dioxide is reduced to CO or CH₄ which is a high value-added material.

FIG. 2 is a schematic diagram showing a method for capturing and photo-converting carbon dioxide in the atmosphere according to an embodiment of the present disclosure.

Referring to FIG. 2, the method for capturing and photo-converting carbon dioxide in the atmosphere according to an embodiment of the present disclosure may include a first step of selectively capturing carbon dioxide in the atmosphere using the photocatalyst of the present disclosure, and a second step of irradiating light to the photocatalyst having the carbon dioxide captured thereon to reduce the captured carbon dioxide to a photo-conversion product.

The first step is a step of selectively capturing low concentration carbon dioxide in the atmosphere using the photocatalyst of the present disclosure. The first step may be performed in a dark condition. When the photocatalyst of the present disclosure is used, carbon dioxide may be well adsorbed thereon even in a general atmospheric environment containing oxygen and moisture and containing carbon dioxide of a low concentration (about 450 ppm).

The second step is a step of irradiating light to the photocatalyst having the carbon dioxide captured thereon to reduce the captured carbon dioxide to a photo-conversion product. In this regard, the light irradiation may include irradiating of sunlight or light having the same or similar wavelength as or to that of the sunlight. For example, the light may be irradiated thereto under an AM 1.5 (100 mW cm⁻²) condition. In one example, carbon dioxide may be reduced to the photo-conversion product including CO or CH₄ upon the irradiation of the light thereto.

According to the present disclosure, the low concentration (about 450 ppm) carbon dioxide in the atmosphere may be selectively adsorbed in a dark condition (night) including oxygen and moisture, and the adsorbed carbon dioxide may be photo-converted in the light-containing condition (day) such that the carbon dioxide may be reduced to a high value-added material.

Hereinafter, Examples of the present disclosure will be described in detail. However, Examples as described below are only some embodiments of the present disclosure, and the scope of the present disclosure is not limited to the following Examples.

### Example 1: Preparation of Bismuth Oxyhalide (Pure BiOX(X=Cl, Br, I))

Bi(NO₃)₃·5H₂O(5 mmol) is put in 50 mL of distilled water (DI water) and completely dissolved through sonication for 10 minutes or greater. 5 mmol of NaX (X=Cl, Br, I) is added thereto, followed by stirring for 30 minutes. The completely dissolved solution is placed into a 100 mL PTFE liner and is hydrothermally synthesized at 160°C for 16 hours. After the reaction is over, the reaction product is cooled to room temperature, was washed several times with water and ethanol. Powders are obtained therefrom via centrifugation, are dried in an oven at 60°C for at least 12 hours.

### Example 2: Preparation of Bismuth Oxyhalide Activated with Oxygen Vacancy (Ov BiOX)

Bi(NO₃)₃·5H₂O(5 mmol) is put in 50 mL of an ethylene glycol solution and completely dissolved through sonication for 10 minutes or greater. 5 mmol of NaX (X=Cl, Br, I) is added thereto, followed by stirring for 30 minutes. 0 to 2 mL of 1M HX (X=Cl, Br, I) is added thereto, followed by stirring for 5 minutes. The completely dissolved solution is placed in a 100 mL PTFE liner and is hydrothermally synthesized at 160°C for 16 hours. After the reaction is over, the reaction product is cooled to room temperature, was washed several times with water and ethanol. Powders are obtained therefrom via centrifugation, are dried in an oven at 60°C for at least 12 hours. The dried powder is heat-treated at 300°C (10° C/min.) for 1 hour in an Ar atmosphere to remove a trace amount of an organic material.

### Example 3: Preparation of Bismuth Oxyhalide having Metal Bonded thereto and Activated with Oxygen Vacancy (M/Ov BiOX)

To bond the metal to the Ov BiOX photocatalyst as prepared according to Example 2, 400 mg Ov BiOX is placed in a PTFE beaker and 60 mL of distilled water was added thereto. A metal precursor is added thereto at 0.01 to 3 wt%, and the beaker is immersed in a 90°C preheated constant temperature water bath, followed by stirring for 1 hour and 30 minutes. After the reaction is completed, the reaction product is cooled to room temperature, is washed with water, is subject to separation using a centrifuge, and is dried in an oven at 60°C for at least 12 hours.

### [Example 4: Preparation of Bismuth Oxyhalide having Two Metals Bonded thereto and Activated with Oxygen Vacancy (M₁M₂/Ov BiOX)]

To bond another metal to the M/Ov BiOX photocatalyst as prepared according to Example 3, 400 mg M/Ov BiOX is placed in a PTFE beaker and 60 mL of distilled water is added thereto. The metal precursor is added thereto at 0.01 to 3 wt%, and the beaker immersed in a 90°C preheated constant temperature water bath, followed by stirring for 1 hour and 30 minutes. After the reaction is completed, the reaction product is cooled to room temperature, is washed with water, is subject to separation using a centrifuge, and is dried in an oven at 60°C for at least 12 hours.

### Experimental Example 1: Evaluation of Photocatalyst Characteristics

In order to analyze the crystal structure of each of the photocatalysts according to Examples 1 to 4, EMPUREAN equipment from the PANalytical company was used to apply Cu-Kα radiation (40 kV, 30 mA) having a wavelength of 1.5406 Å to powders of Pure BiOCl (Example 1), Ov BiOCl (Example 2) and CuFe/Ov BiOCl (Example 4) for measurement thereof.

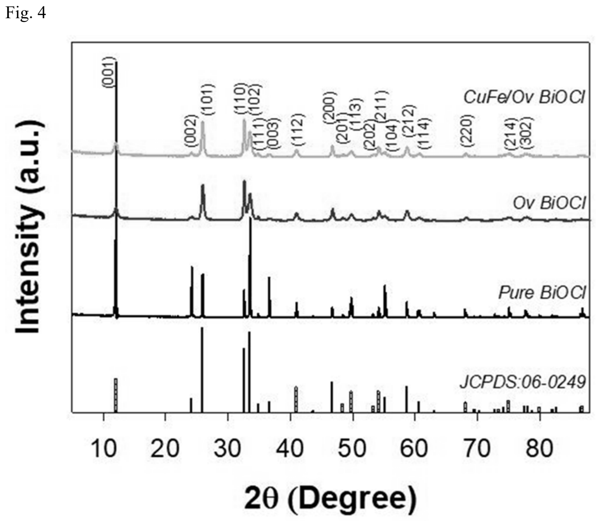

Referring to FIG. 4 showing the result, it may be identified that the crystal structure of the pure BiOCl photocatalyst is identical with that of BiOCl (JCPDS 06-0249). The diffraction peak of Ov-BiOCl activated with the oxygen vacancy exhibited a lower intensity and a wider peak width than those of the pure BiOCl photocatalyst, thereby exhibiting a broadened pattern. In addition, CuFe/Ov BiOCl carrying CuFe ions did not significantly change in XRD.

On the other hand, in order to measure the composition and components of the photocatalyst surface, ThermoFisher (NEXSA) equipment was used to apply Al-Kα (1486.6 eV) to the powders of Pure BiOCl (Example 1), Ov BiOCl (Example 2) and CuFe/Ov BiOCl (Example 4) for measurement thereof. The surface composition of the prepared sample was analyzed. This indicated that the composition includes the elements Bi, Cl, and O. In CuFe/Ov BiOCl, presence of the elements Cu and Fe was confirmed.

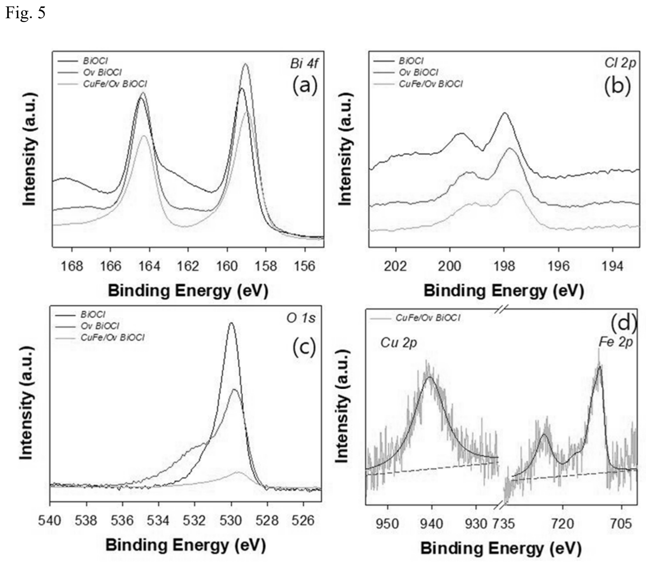

Referring to FIG. 5 showing the result, when the Bi 4f peaks were compared with each other first, the peaks of 159.2 and 164.5 eV correspond to two peaks of Bi 4f_{7/2} and Bi 4f_{5/2}. In the Ov BiOCl activated with the oxygen vacancy, 159.2 and 164.5 eV shifted to the low binding energy (159 and 164.3 eV) by 0.2 eV. This is a phenomenon caused by Bi^{(3-x)+} formation due to the oxygen deletion. Two peaks belonging to Cl 2p_{3/2} and Cl 2p₁₂ were also identified in the Cl 2p spectrum. The new peak appearing at a higher binding energy relative to the O 1s peak of Ov BiOCl may be due to oxygen coordination, such as OH or adsorbed oxygen. This also suggests the presence of the oxygen deletion. It may be clearly identified that the contribution of Bi-O is reduced in the BiOCl having Ov generated via the removal of the oxygen atom binding to the Bi atom. In (d) in FIG. 5, Cu and Fe ions were well supported on CuFe/Ov BiOCl, and thus the presence of the elements Cu and Fe ions was identified.

Next, the change in the color of the photocatalyst after the surface modification of the photocatalyst was observed. The wavelength range of the light as absorbed by the photocatalyst was measured using an analyzer manufactured by the Shimadzu company. BaSO₄ (Barium sulfate) was used as a reference. The light absorption spectrum of the photocatalyst powders regarding the light of the wavelength range from 200 nm to 900 nm was measured based on the refence.

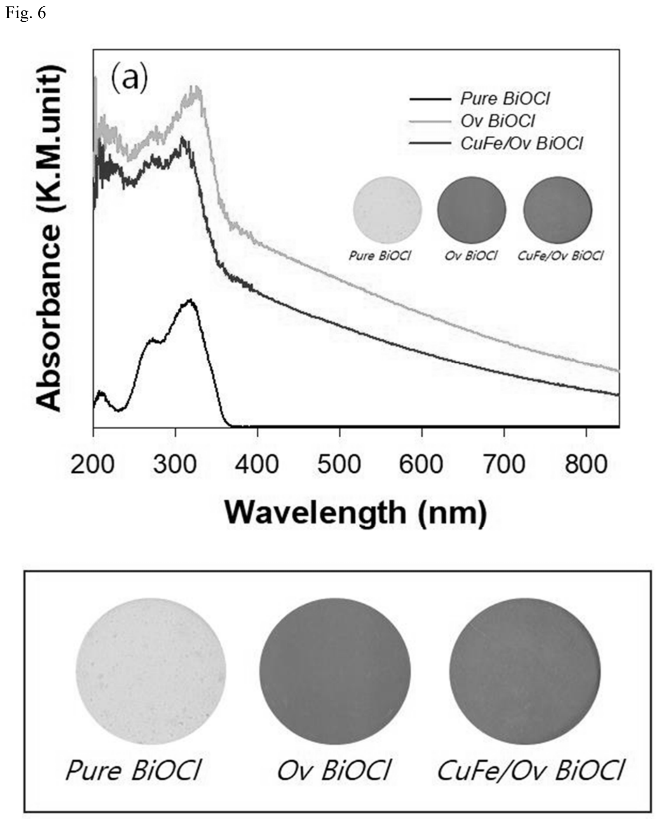

As a result, as shown in the photocatalyst color of FIG. 6, the color of the photocatalyst was changed to dark brown (Ov BiOCl) due to the activation with the oxygen vacancy. In addition, as shown in DRS, the absorption of the visible light and infrared wavelength bands of the photocatalyst activated with the oxygen vacancy was significantly increased compared to the conventional BiOCl. The photocatalyst carrying CuFe ions thereon exhibited no significant difference in the photocatalyst color and DRS from those of Ov BiOCl.

On the other hand, CuFe/Ov BiOCl sample powders were measured using a field emission transmission electron microscope FE-TEM (200 kV) for image observation, component and structure analysis of the ultra-fine regions of the photocatalysts according to Examples 1 to 4.

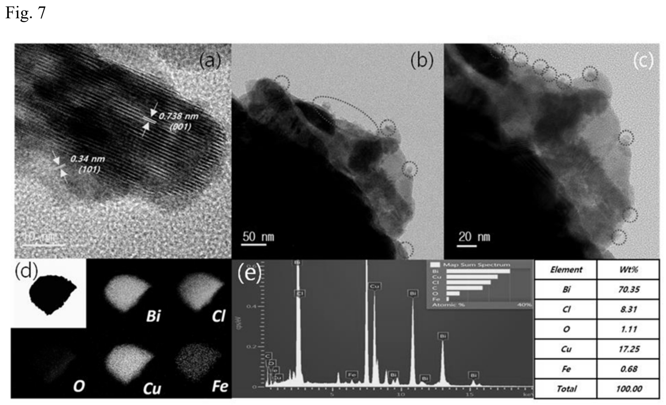

As a result, (001) and (101) lattices of BiOCl were also identified in TEM analysis as in the crystal phase as identified in XRD ((a) in FIG. 7). In addition, it was identified that the metal cluster shape was attached to the surface in (b) and (c) in FIG. 7. The presence or absence of Bi, O, Cl, Cu, and Fe elements was identified via EDS analysis, and the BiOCl photocatalyst was observed via TEM. Thus, it may be identified that Cu and Fe were well supported thereon.

### Experimental Example 2: Experiment of Adsorption and Conversion of Carbon Dioxide in Atmosphere

In order to evaluate the adsorption and conversion performance of carbon dioxide in the atmosphere, about 0.05% carbon dioxide (CO₂)/air atmosphere (Air Balance) as an actual atmospheric condition was used. 150 mg of the photocatalyst according to Example 4 was input into a 3 cm² wide sample holder which in turn was placed on a bottom of a stainless steel reactor of about 120 mL. Air was injected into the reactor for 12 hours at 200°C for surface pretreatment (carbon and organic material removal). The concentration of each of carbon dioxide and air was adjusted by controlling the flow rate of each of carbon dioxide and air using a flow controller (MFC). The carbon dioxide and air flowed through an impinger containing water such that the humidity was maintained at ±45%. The mixed gas was put in a sampling gas bag in advance, and the pretreatment thereon was completed, and then the inside of the reactor was brought into a vacuum state. The inside of the reactor was filled with the mixed gas using the gas bag containing the mixed gas therein.

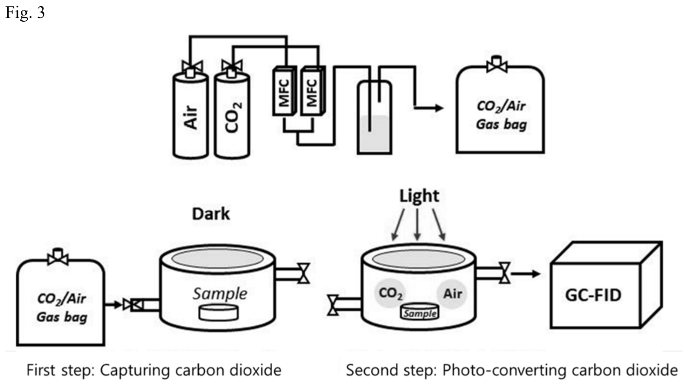

The carbon dioxide was captured for 2 hours and change in the concentration of the captured carbon dioxide was checked, and a light source (solar light, AM 1.5, 100 mW cm⁻²) was applied thereto to sample the gas in the reactor for a certain period of time to check the material as generated and an amount thereof. As the gas, GC-FID (YL) was used to check the decomposition and production amounts by quantification of each of carbon monoxide and carbon dioxide. (See FIG. 3)

### Experimental Example 3: Results of Experiment on Adsorption and Conversion of Carbon Dioxide in Atmosphere

### Comparison of Capture and Conversion of Carbon Dioxide in Atmosphere of Pure BiOCl and Ov BiOCl Photocatalysts

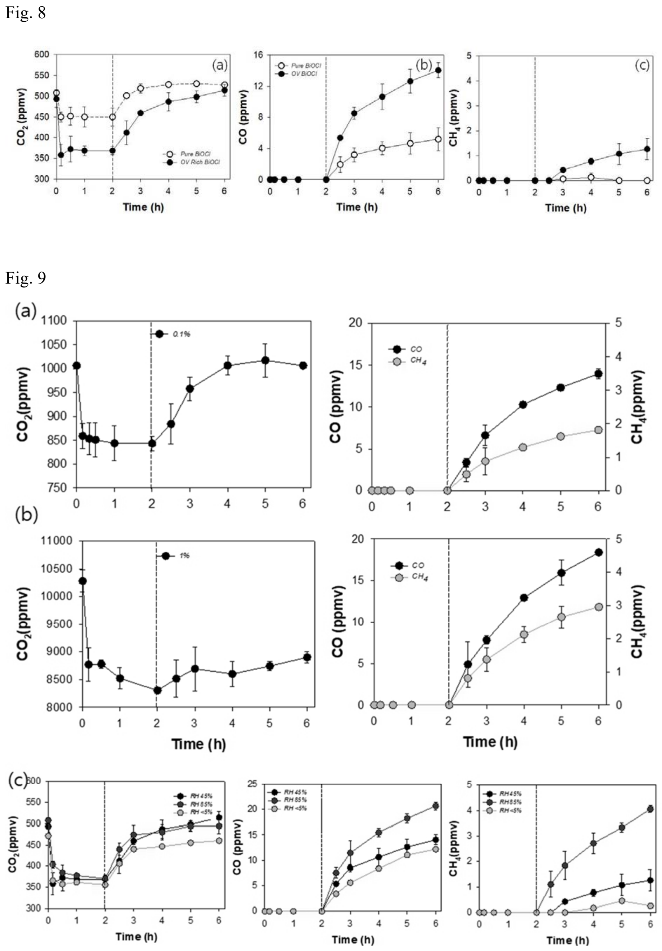

FIG. 8 shows the results of capture and conversion of carbon dioxide in the atmosphere of Pure BiOCl photocatalyst and the Ov BiOCl photocatalyst activated with oxygen vacancies. (a) in FIG. 8 shows the result of capturing carbon dioxide at about 0.05% carbon dioxide condition in an air atmosphere containing oxygen similar to the carbon dioxide condition in the atmosphere, and (b) and (c) in FIG. 8 respectively show the concentrations of carbon monoxide and methane as the products obtained by photo-converting the captured carbon dioxide using sunlight.

As a result, the capture rate of the carbon dioxide in the atmosphere of the photocatalyst activated with the oxygen vacancy compared to the Pure BiOCl was 150 ppmv, which increased to about three times of that of the Pure BiOCl. It was identified that the photocatalyst activated with the oxygen vacancy selectively adsorbed the carbon dioxide relatively better due to being activated with the oxygen vacancy. In addition, an amount of the photo-conversion product of the captured carbon dioxide obtained by the photocatalyst of Ov BiOCl is greater than three times of that of the Pure BiOCl.

### Evaluation of Capture Performance of Ov BiOCl based on Carbon Dioxide Concentration in atmosphere and Humidity Conditions

FIG. 9 is a result of evaluating the capture performance of the Ov BiOCl photocatalyst of carbon dioxide at the concentration of carbon dioxide (0.05 to 1%) in the atmosphere. As a result, about 250 ppmv of the capture rate at 0.1% (1000 ppmv) of the carbon dioxide was identified. The photo-conversion products as CO and CH₄ were produced at 15 ppmv and 2 ppmv, respectively, at 0.1% (1000 ppmv) of carbon dioxide. In addition, it was identified that at a 1% (10000 ppmv) carbon dioxide concentration, about 1200 ppmv capture rate was achieved, and the photo-conversion products as CO and CH₄ were produced at 18 ppmv and 3 ppmv, respectively.

The difference between the capture rates based on the concentrations of carbon dioxide in the atmosphere and the difference between the photo-conversion product amounts based on the concentrations of carbon dioxide in the atmosphere were analyzed above, and as a result, it was identified that the capture performance and the photo-conversion performance tended to increase as the concentration of carbon dioxide in the atmosphere increased.

Next, the capturing and converting performance of the carbon dioxide in the atmosphere based on the humidity was evaluated. As a result, there was no significant difference in the amount of carbon dioxide as captured based on the varying humidity. The selective adsorption of the carbon dioxide of the photocatalyst is not affected in competition with water in terms of the adsorption of the carbon dioxide. However, in the photo-conversion performance, the concentration of each of the products as CO and CH₄ was the highest at 85% of RH with high moisture, which was due to the difference caused by the H produced on the surface of the photocatalyst as the water was dissociated.

### Comparison of Capture and Conversion Performance of M/Ov BiOCl of Carbon Dioxide in Atmosphere

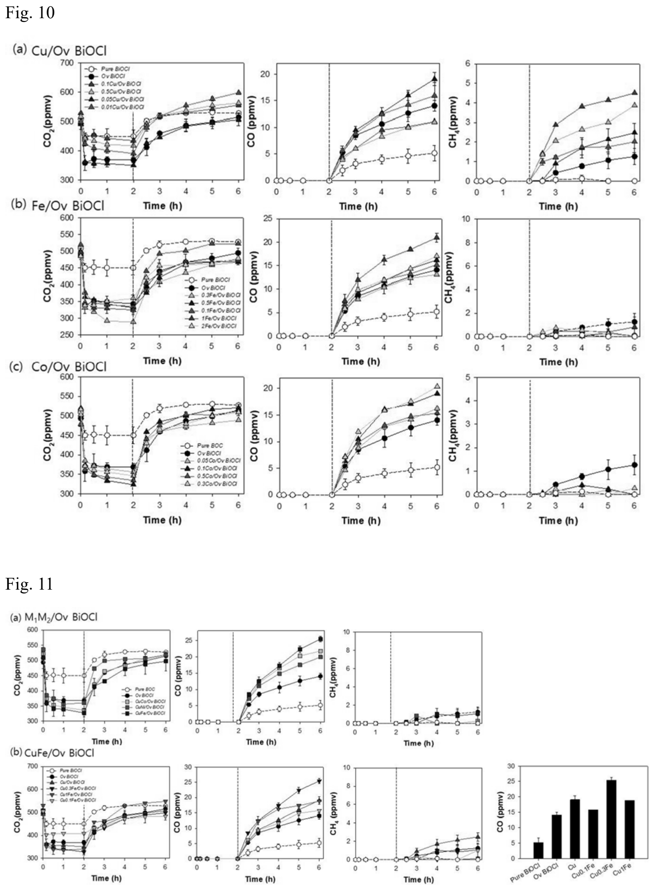

(a) to (c) in FIG. 10 shows the results of comparing the carbon dioxide capture and conversion performances of the photocatalysts in which Cu, Fe, and Co are respectively supported on the Ov BiOCl photocatalyst. Various metals were supported thereon at the content of 0.01 to 2 wt% and then the performances of the photocatalysts on which the various metals were respectively supported were compared with each other. Thus, the difference between carbon dioxide capture and photo-conversion performances thereof based on the type of the metal supported thereon was identified.

As the metal is supported on the photocatalyst, the amount of the carbon dioxide captured thereby was additionally increased to about 30 to 50 ppmv compared to the existing Pure BiOCl and Ov BiOCl. The photo-conversion performance of the photocatalyst having the metal supported thereon was improved by about 4 times or greater compared to that of the Pure BiOCl. The carbon dioxide capture and conversion performance was optimized at each of 0.05 wt% Cu, 1 wt% Fe, and 0.3 wt% Co.

### Comparison of Capture and Conversion Performance of M₁M₂/Ov BiOCl of Carbon Dioxide in Atmosphere

(a) in FIG. 11 shows a result obtained by comparing the carbon dioxide capture and conversion performances of the bimetallic photocatalysts M₁M₂/Ov BiOCl (M₁M₂=CuCo, CuNi, CuFe) with each other. As a result, the photocatalyst having CuFe supported thereon exhibited the best carbon dioxide capture and photo-conversion performance. The capture performance thereof was additionally improved by 160 ppmv and 100 ppmv compared to Pure BiOCl and Ov BiOCl, respectively. The photo-conversion performance thereof was improved by about 5 times and 2.5 times, compared to Pure BiOCl and Ov BiOCl, respectively. As described above, it was identified that when Cu+Co, Cu+Ni, Cu+Fe, or the like was supported thereon, the performance thereof was increased compared to the existing photocatalyst.

(b) in FIG. 11 shows a result of evaluating the carbon dioxide capture and conversion performance when 0.05 wt% Cu having the best performance is fixed and Fe is added at a varying content in order to optimize the content of each of the finally selected Cu and Fe. In this regard, the performance was the best when 0.05 wt% Cu and 0.3 wt% Fe was selected.

### Capture and Conversion Performance of Optimized Catalyst of Carbon Dioxide in Atmosphere

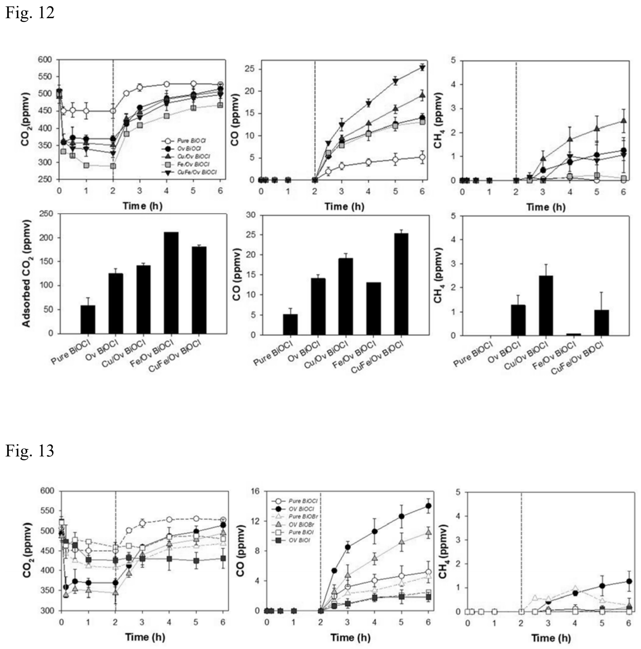

FIG. 12 shows the capture and conversion performances of the optimized photocatalysts of carbon dioxide in the atmosphere. Referring to FIG. 12, Fe/Ov BiOCl containing 1 wt% of Fe exhibited the highest carbon dioxide capture performance, followed by CuFe/Ov BiOCl having 0.05 wt% Cu and 0.3 wt% Fe exhibiting the high carbon dioxide capture performance.

The photo-conversion performance of the bimetallic photocatalyst (CuFe/Ov BiOCl) was significantly higher than that of each of Fe/Ov BiOCl and Cu/Ov BiOCl.

### Comparison of Capture and Conversion Performances of Pure BiOX and Ov BiOX (X=Cl, Br, I) of Carbon Dioxide in Atmosphere

FIG. 13 shows the capture and conversion performance of the carbon dioxide in the atmosphere using the photocatalyst in which a type of a halogen element is varying. Referring to FIG. 13, all of the photocatalysts respectively containing Cl, Br, and I exhibited the capture and conversion performance of the carbon dioxide in the atmosphere.

Although the present disclosure has been described above with reference to the preferred embodiments of the present disclosure, those skilled in the art will understand that the present disclosure may be variously modified and changed within the scope not departing from the spirit and scope of the present disclosure as described in the following patent claims.

## Claims

1. A photocatalyst for capturing and converting carbon dioxide, the photocatalyst comprising a bismuth oxyhalide having at least one metal supported thereon and having an oxygen vacancy.

2. The photocatalyst for capturing and converting carbon dioxide of claim 1, wherein the metal is selected from Cu, Fe, Co, Ni, Mn, Ru, Pt, Au, and Ag.

3. The photocatalyst for capturing and converting carbon dioxide of claim 1, wherein the metal is contained at a content of 0.01 to 2 wt% based on a total weight of the photocatalyst.

4. The photocatalyst for capturing and converting carbon dioxide of claim 1, wherein the metal includes Cu and Fe.

5. The photocatalyst for capturing and converting carbon dioxide of claim 4, wherein Cu is contained at a content of 0.01 to 0.5 wt% based on a total weight of the photocatalyst, and Fe is contained at a content of 0.3 to 1 wt% based on the total weight of the photocatalyst.

6. The photocatalyst for capturing and converting carbon dioxide of claim 1, wherein the photocatalyst selectively captures carbon dioxide having a concentration of 1000 ppm or lower in the atmosphere under a dark condition.

7. The photocatalyst for capturing and converting carbon dioxide of claim 6, wherein the photocatalyst converts the captured carbon dioxide into CO or CH₄ upon irradiation of light thereto.

8. A method for capturing and photo-converting carbon dioxide in the atmosphere, the method comprising:
a first step of selectively capturing carbon dioxide in the atmosphere, using the photocatalyst according to one of claims 1 to 7; and
a second step of irradiating light to the photocatalyst having the carbon dioxide captured thereon to reduce the captured carbon dioxide to a photo-conversion product.

9. The method for capturing and photo-converting carbon dioxide in the atmosphere of claim 8, wherein the first step is performed under a dark condition.

10. The photocatalyst for capturing and converting carbon dioxide in the atmosphere of claim 8, wherein the photo-conversion product includes CO or CH₄.
